# EUROPEAN PATENT APPLICATION

(11) **EP 3 560 421 A1**
(43) Date of publication of application: **30.10.2019**
(21) Application number: 18460064.1
(22) Date of filing: 06.11.2018
(51) Int. Cl.: A61B 5/0402, A61B 5/0408, A61B 5/00

(54) **THE FLEXIBLE MEASURING IMPLANT**

(30) Priority: 26.04.2018 PL 42537418
(71) Applicant: Invis sp. z o.o., 02-032 Warszawa (PL)
(72) Inventor: KACZMAREK, Piotr, 60-651 Poznan (PL); LAMPRECHT, Patryk, 05-120 Legionowo (PL)
(74) Representative: Dziubinska, Joanna

(57) **Abstract**

The flexible measuring implant is characterized by the fact that it has at least two electrodes (2) placed one on the outside of the strap and the other on the side of the strap in contact with the forearm and has a set of amplifiers (8) and analog filters (9) of the ECG signal, an analog-digital converter serving for conversion of analog signal to digital form, module for wireless digital transmission of ECG signal; wherein the analog filter block (9) comprises at least an anti-aliasing low-pass filter for removing higher frequencies from the signal; a flexible measuring insert transmits digital data to the bioidentification module, whose task is to perform user authorization based on the implemented bioidentification algorithm; and the bioidentification module can be implemented in a flexible measuring implant or in a master device (e.g. a mobile phone) with which the device cooperates.

## Description

The subject of the application is a flexible measuring implant for use in fashion elements such as watch strap, bracelet, band.

The device is a flexible element with an internal electronic-mechanical systems, placed there for controlling electronic devices, in particular mobile phones, tablets, PDA (personal digital assistant), intelligent home appliances, cars, etc. The flexible remote communication implant according to the invention is waterproof.

The flexible measuring implant according to the invention has the ability to measure ECG and bioidentification of the user.

From the description of the utility model No. W 123727 is known the "Housing of the wrist band of monitoring of vital signs", which has a two-element construction. The main part of the casing is made of flexible plastic, in which there is a socket for a plastic casing for electronic components. On the top layer of the housing there is a convex SOS button with the word in Braille. In the lower part of the housing, which adheres to the wrist, there is an optical sensor and a thermal sensor. A strip with a placed membrane button and a RGB LED diode is led out of the plastic housing. The plastic housing is blocked by flexible splines.

From the description of the invention No. P 418185 is known a "system consisting of the device and methods of discreet informing about adverse cardiovascular events or falls and a method of two-way communication between the user and the supervisor", which includes a sensor worn on the user's hand, a receiver belonging to the monitoring person and the server, where the sensor monitors the biological parameters of the user or describes its movement thanks to built-in sensors: accelerometer, gyroscope, magnetometer, pulse oximeter, SIM module (GSM), GPS receiver and electrodes, also having an alarm button in the form of the surface of the watch, which includes analogue watch glass and watch face, and in the case of a digital watch, the sensor is characterized by the fact that the sensor connects directly to the receiver, sending an alarm in the form of a text message or sound or image or video, using a mobile network or the Internet.
From the description of the invention No. P 407387 is known "Integrated measuring and testing device", which is made in the form of goggles. The housing contains measuring heads with IR illuminators and IR filters as well as side cameras with an adjustable position that observe the image of the eye reflected from the infrared mirrors. The measuring heads have removable covers. On the top surface of the goggle housing, the front camera is mounted, the cameras transmit the image directly to the computer system, and the side cameras are coupled with configurable illuminators controlled by a digital controller and equipped with a set of three-color LEDs. The front camera is obscured by an IR filter allowing only infrared light. In addition, there are at least one IR diode located under the IR filter, at least one RGB-LED, one RGB-LED, one red LED, placed above the IR filter and photodiode and all LEDs and IR have adjustable brightness.

The essence of the flexible measuring insert according to the invention is that it has at least two electrodes located one on the outside of the strap and the other on the side of the strap in contact with the forearm and has a set of amplifiers and analogue ECG filters, an analog-digital converter used to convert the signal analogue to digital form, module for wireless digital transmission of ECG signal. The block of analog filters consists of at least an anti-aliasing low-pass filter to remove higher frequencies from the signal. The flexible measuring implant transmits digital data to the bioidentification module, whose task is to perform user authorization based on the implemented bioidentification algorithm; wherein the bioidentification module can be implemented in a flexible measuring implant or in a master device (e.g. a cell phone) with which the device cooperates.

The implant preferably has a PCB with a control unit and sensors, a battery, a vibration motor or a loudspeaker, a charging connector and a charger charger connector or a wireless charging antenna, an NFC antenna with a chip. The PCB inside the flexible implant can be in an elastic form, using an elastic or rigid laminate using a rigid laminate, and can also be made with a combination of these two technologies - a rigid-flexible laminate. In a particular case, the connection between the rigid elements is flexible e.g. by a flexible tape or a wired connection.

All elements of the electronic system have proper arrangement and mechanical properties that ensure the possibility of placing the device in the implant and the subsequent construction of the watch strap, ensuring its natural size and flexibility. Consumer electronics placed in a flexible implant can be used, among others, for communication with mobile devices or for contactless payments, entry exit security systems, loyalty cards, identity card, patient card, sports training monitoring, user's biometric functions, communication functions, gesture detection and their combinations.

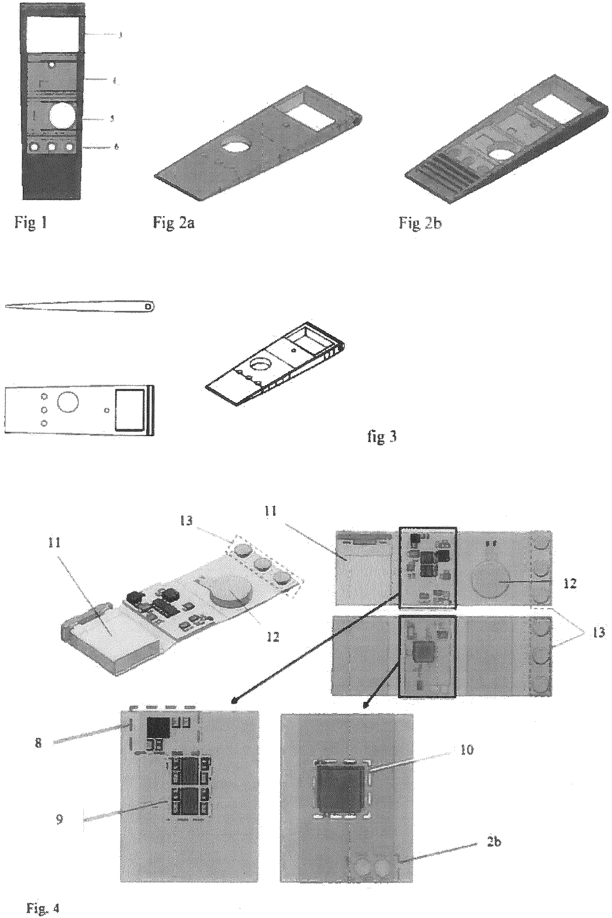

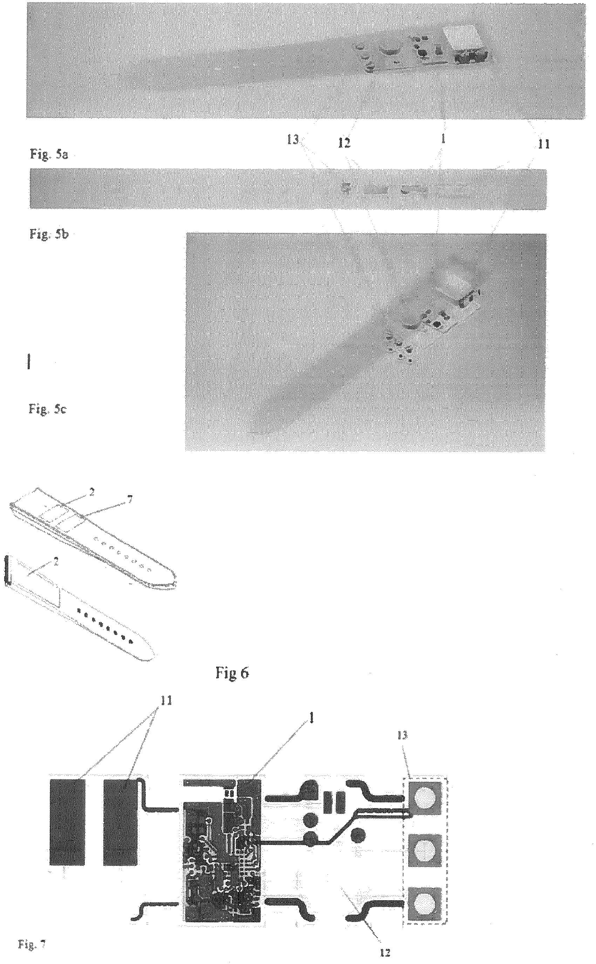

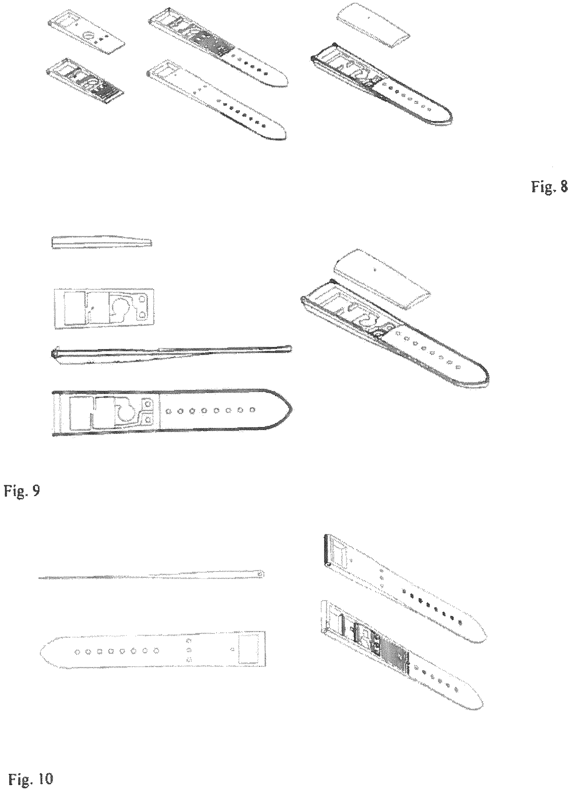

The solution according to the invention is shown in the attached drawing, figures 1-10, where:
Fig. 1 is a schematic diagram showing the arrangement of electronic components of a part of watch strap being a casing of a flexible implant according to the invention.
Fig. 2a shows a part of the watch strap being a casing of a flexible implant according to the invention - top view.
Fig. 2b shows a part of the watch strap being a casing of a flexible implant according to the invention - bottom view.
Fig. 3 shows a side, top and perspective view of a part of the watch strap being a casing of a flexible implant according to the invention.
Fig. 4 shows an electronic component with details of a flexible implant according to the invention.
Figs. 5a, 5b and 5c show the position of the elastic implant according to the invention in the watch strap.
Fig. 6 shows the position of the electrodes being an element of the elastic implant according to the invention on the surface of the watch strap.
Fig. 7 shows a diagram of the electronics used in the elastic implant according to the invention.
Fig. 8 shows the possibilities of providing a flexible casing for a flexible implant according to the invention in a short, long version and a hybrid strap.
Fig. 9 shows a side view from above and a perspective view of a part of a watch strap being a long version of a flexible implant casing according to the invention.
Fig. 10 shows a side, top and perspective view of a part of the watch strap being a casing of a flexible implant according to the invention in a hybrid strap version.

The solution according to the invention is shown in the non-limiting embodiment of the invention:
The flexible ECG signal implant is embedded in a watch strap, in which a flexible implant containing a PCB (1), a battery, electrodes (2) for archiving the ECG signal is placed. The implant according to the invention has at least two electrodes (2) of which at least one is located on the outer - side of the strap and at least one on the other side of the strap - on the surface on the side of the forearm. The electrodes (2) through the places (2b) of soldering the electrode signal wires, transmit the ECG signal to the subsequent parts of the device according to the invention.
The electrodes used in the device can be made in the form of a fabric made of conductive fibers, a flexible conductive polymer, or they can be capacitive electrodes located under the surface of the skin of the watch strap.

The flexible measuring implant according to the invention has the following elements integrated: a set of amplifiers (8) and analog filters (9) of the ECG signal, an analog-digital converter used to convert an analog signal into a digital form, a module for wireless digital transmission of the ECG signal. In the solution according to the invention, the electrodes (2) through wires are connected to an amplifier (8) which transmits the signal to the analog filter (9). The signal through the transducer is transmitted to the wireless data transmission module.

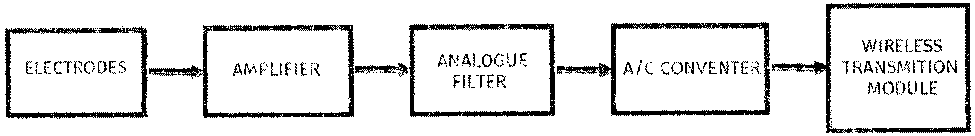

The amplifier module (8) of the patient's ECG signal consists of buffer amplifiers with gain times between 1-200 and, optionally, a differential amplifier, on the input signals from the leads (2). The third electrode (Reference / DRL) (7) can be used to compensate for existing patient interferences. The analog filter block (9) consists of at least an anti-aliasing low-pass filter that cuts off frequencies higher than X (X - a value between 100 - 1000 Hz not more than half of the analog-to-digital converter sampling frequency). In a preferred solution, a bandpass filter cutting out the 50Hz frequency and an upper-pass filter cutting the signal components at frequencies lower than Y (Y - value between 0-5 Hz) can be used to eliminate the signal constant component. In a variation of the invention, a digital filter may be used.

The flexible implant can use an analog-digital converter built into the microcontroller (10) or in an external circuit. The wireless data transmission module can be a standalone system or be integrated with a microcontroller (10). The flexible implant may include a processor for digital signal processing. The flexible implant sends digital data to the bioidentification module, whose task is to perform user authorization based on the implemented bioidentification algorithm. The module may be implemented in a device according to the invention or in a master device (e.g. a mobile phone) with which the device cooperates.

The flexible measuring implant has an amplifier module (8) of the ECG signal from the patient. It consists of buffer amplifiers, to which the signal is transmitted through soldered wires (2) on the PCB (2b), and the third electrode (DRL) (7) is used to compensate for already existing disturbances on the patient and as a reference voltage source.

To use the solution according to the invention, the user applies a finger or fingers on which there is no watch / bracelet with a built-in flexible measuring insert for the electrode or electrodes (2) located on the outside of the strap. Then the device starts recording the ECG signal and converts it into a digital form. The ECG signal is sent to the bioidentification module, which as a result of the operation provides information about the positive or negative result of the process.

In the solution according to the invention, the measurement system monitors the relationship between the current and voltage in the circuit between the electrodes, which allows the automatic detection of a second hand finger application. When the finger is not applied, most of the processing path circuits remain in sleep mode. After applying the finger, the processing path is woken up and the signal acquisition begins. The signal is recorded by means of a pair of electrodes, it is amplified and filtered, an analog low-pass filter and an analog or digital highpass filter. The analog-to-digital converter converts the voltages into digital form. The received digital data is sent to the mobile device for further processing.

The preprocessing algorithm performs the following actions:
- filtering and monitoring of power grid disturbances
- detection of PQRST ECG complexes
- approximation of detected PQRST complexes using a spline model
- determination of signal quality parameters in the form of SNR and electrode-skin contact impedance

In this solution, a vector of numerical features is extracted based on the characteristics of individual waves (e. g. duration of the wave, amplitude of individual waves).
The elastic insert according to the invention performs two tasks: it measures the ECG to build the ECG signal model of the user and measures the ECG to authorize the user. In the first case, the ECG signal is recorded several times in sequences enabling the recording of at least 5 PQRST waves. The registered features of each sequence are used to build a given user model with parameters estimated using machine learning methods. In functional terms, the model is a classifier whose entry is a feature vector and the output is the probability that a given feature vector originates from a particular user.
In the second case, the feature vector determined for the ECG waveform containing at least 5 PQRST complexes of a given user is given to the entry of the classifier using the ECG model of a given user. On the basis of the probability value of belonging of a feature vector to a given user, discrimination of this value against a certain threshold is realized. When the probability is greater than the threshold, the authorization returns a positive result, otherwise the result is negative.

In the case of negative authorization, the user has the possibility to carry out the authorization in a different way (e. g. by entering the PIN number on the mobile device). In this case, the user model is traversed and the incorrectly categorized feature vector is placed in the learning sequence. This situation also occurs when the authorization gave a positive result, however the registered feature vector is poorly conditioned in the currently used model.

The implant according to the invention for a strap, e.g. a watch or a bracelet, allows the electronics to be placed in its interior. It has a specially designed place to implement in accordance with the shape. It consists of two elements - base and cover.

The device for measuring the ECG signal is embedded in the watch strap, which can be integrated with any watch case with a lugs' width limited by the width of the strap and is placed on the forearm. A flexible implant containing a PCB (1) is placed in any watch strap (in a preferred rigid-flex option [Rigid-Flex] PCB), a battery (11), electrodes for archiving the ECG signal (2).

The watch strap, in which the elastic implant is placed, according to the invention has an internal structure composed of elements (3), (4), (5), 6) preferably rigid. The individual elements (3), (4), (5), 6) can have different lengths. These elements are openings for locating the electronic part of the device according to the invention in a flexible casing. Preferably, each subsequent element is shorter by at least 1 mm from the previous element. The distance between consecutive elements (3), (4), (5), 6) should preferably have a minimum of 1 mm required due to the reserve of movement of the elements during bending. The height of the next elements / holes (3), (4), (5), 6) should decrease. Each subsequent element (3), (4), (5), 6) should be smaller than the preceding element.

The solution according to the invention has at least two electrodes (2) of which at least one is located on the outside of the strap and at least one on the strap side in contact with forearm. The electrodes used in the device can be made in the form of a fabric made of conductive fibers, a flexible conductive polymer, or they can be capacitive electrodes located under the surface of the skin of the watch strap.

The solution according to the invention has the following elements integrated: a set of amplifiers (8) and analog filters (9) of the ECG signal, an analog-digital converter used to convert an analog signal into a digital form, a module for wireless digital transmission of an ECG signal. The device according to the invention is powered by a battery (11). The rigid part of the PCB (1) is connected to the battery (11) and the device's charging connectors (13). Preferably, the connection is made by a flexible laminate connected to a rigid PCB (1).

In a variant of the invention, the elastic casing of the elastic implant according to the invention can be made in a short, long version and a hybrid strap. In a variant of the invention, the device may have a vibration motor (12) to inform the user about selected events.

## Claims

1. A flexible measuring insert containing an implant cooperating with a mobile application, at least one electronic circuit, a power element, a charging module **characterized in that** it has at least two electrodes (2) located one on the outside of the strap and the other on the strap side in contact with forearm and has a set of amplifiers (8) and analog filters (9) of the ECG signal, an analog-digital converter used to convert an analog signal into a digital form, a module for wireless digital transmission of an ECG signal.

2. A flexible measuring insert according to claim 1, **characterized by** a block of analog filters (9) consists of at least an anti-aliasing low-pass filter to remove higher frequencies from the signal.

3. A flexible measuring insert according to claim 1, **characterized in that** it transmits digital data to the bioidentification module, whose task is to perform user authorization based on the implemented bioidentification algorithm.

4. A flexible measuring insert according to claim 3, **characterized in that** the bioidentification module may be implemented in a flexible measuring implant or in a master device (e.g. a mobile phone) with which the device cooperates.
